# EUROPEAN PATENT SPECIFICATION

(11) **EP 1 099 759 B1**
(45) Date of publication and mention of the grant of the patent: **20.02.2008**
(21) Application number: 98964347.3
(22) Date of filing: 30.12.1998
(51) Int. Cl.: C12N 1/21, A61K 39/106

(54) **VIBRIO CHOLERAE VACCINE CANDIDATES AND METHODS OF THEIR CONSTRUCTING**
VIBRIO CHOLERAE IMPFSTOFFKANDIDATEN UND VERFAHREN ZU DEREN HERSTELLUNG
NOUVEAUX CANDIDATS VACCINS CONTRE LE VIBRIO CHOLERAE ET LEUR PROCEDE D'OBTENTION

(30) Priority: 30.12.1997 CU 14297
(43) Date of publication of application: 16.05.2001
(73) Proprietor: CENTRO NACIONAL DE INVESTIGACIONES CIENTIFICAS, Ciudad de la Habana 12100 (CU)
(72) Inventor: CAMPOS GOMEZ, Javier, Santa Clara 50100 (CU); FANDO CALZADA, Rafael Alfredo, Ciudad de la Habana 11400 (CU); RODRIGUEZ GONZALEZ, Boris Luis, Ciudad de la Habana 11500 (CU); LEDON PEREZ, Talena Yamile, Ciudad de la Habana 12400 (CU); VALLE DIAZ, Edgar, Camaguey 70800 (CU); SILVA CABRERA, Anisia Juana, Ciudad de la Habana 12100 (CU); BENITEZ ROBLES, Jorge Antonio, Ciudad de la Habana 12100 (CU)
(74) Representative: Braun, André jr.
(86) International application number: PCT/CU1998/000008
(87) International publication number: WO 1999/035271

(56) References cited:
- WO-A-95/18633
- US-A- 5 434 065
- ROBERT A ET AL: "Tagging a Vibrio cholerae El Tor candidate vaccine strain by disruption of its hemagglutinin/protease gene using a novel reporter enzyme: Clostridium thermocellum endoglucanase A" VACCINE, vol. 14, no. 16, 1 November 1996 (1996-11-01), page 1517-1522 XP004063307
- HÄSE, C.C. ET AL.: "Cloning and nucleotide sequence of the Vibrio cholerae hemagglutinin/protease (HA/protease) gene and construction of an HA/protease-negative strain." JOURNAL OF BACTERIOLOGY, vol. 173, no. 11, June 1991 (1991-06), pages 3311-7, XP002104236
- ATTRIDGE, S.R.: "Thymidine auxothophy as an attentuating marker in Vibrio cholerae." MICROBIAL PATHOGENESIS, vol. 19, no. 1, July 1995 (1995-07), pages 11-8, XP002104237
- DATABASE WPI Section Ch, Week 8412 Derwent Publications Ltd., London, GB; Class B04, AN 84-072947 XP002104241 & SU 1 017 726 A (ROST ANTI-CHOLERA), 15 May 1983 (1983-05-15)
- DATABASE EMBL - EMPRO [Online] Entry VCTHYAG, Acc.no. Y17135, 1 May 1998 (1998-05-01) VALLE, E. ET AL.: "Vibrio cholerae thyA gene." XP002104240
- DATABASE EMBL - EMPRO [Online] Entry Vch6514, Acc.no.AJ006514, 5 June 1998 (1998-06-05) CARLIN, N.I.A. ET AL.: "Vibrio cholerae lgt and thyA genes." XP002104239
- BENÍTEZ, J.A. ET AL.: "Preliminary assessment of the safety and immunogenicity of a new CTX-phi-negative, hemagglutinin/protease-defective El Tor strain as a cholera vaccine candidate" INFECTION AND IMMUNITY, vol. 67, no. 2, February 1999 (1999-02), pages 539-45, XP002104238

### SEQUENCE LISTING

<110> Centro Nacional de Investigaciones Cientificas
<120> Novel Vibrio cholerae vaccine candidates and the methods of constructing.
<130> uncertain
<140> PCT/CU 98/00008
   <141> 1998-12-30
<150> CU 142/97
   <151> 1997-12-30
<160> 2
<170> PatentIn Ver. 2.1
<210> 1
   <211> 852
   <212> DNA
   <213> Vibrio cholerae
<220>
   <221> CDS
   <222> (1).(852)
   <223> thyA nucleotide sequence
<400> 1
   gtg aga cag tat tta gat ctt tgt cag cgc atc gtc gat caa ggt gtt    48
   Val Arg Gln Tyr Leu Asp Leu Cys Gln Arg Ile Val Asp Gln Gly Val
     1               5                  10                  15
   tgg gtt gaa aat gaa cga acg ggc aag cgt tgt ttg act gtg att aat    96
   Trp Val Glu Asn Glu Arg Thr Gly Lys Arg Cys Leu Thr Val Ile Asn
                20                  25                  30
   gcc gat ttg acc tac gat gtg ggc aac aat cag ttt cct cta gtc act    144
   Ala Asp Leu Thr Tyr Asp Val Gly Asn Asn Gln Phe Pro Leu Val Thr
            35                  40                  45
   aca cgc aag agt ttt tgg aaa gcc gcc gtg gcc gag ttg ctc ggc tat    192
   Thr Arg Lys Ser Phe Trp Lys Ala Ala Val Ala Glu Leu Leu Gly Tyr
        50                  55                  60
   att cgt ggt tac gat aat gcg gcg gat ttt cgc caa tta ggt acc aaa    240
   Ile Arg Gly Tyr Asp Asn Ala Ala Asp Phe Arg Gln Leu Gly Thr Lys
   65                  70                  75                  80
   acc tgg gat gct aat gcc aat tta aac caa gca tgg ctc aac aat cct    288
   Thr Trp Asp Ala Asn Ala Asn Leu Asn Gln Ala Trp Leu Asn Asn Pro
                   85                  90                  95
   tac cgt aaa ggt gag gat gac atg gga cgc gtg tat gga gtt cag ggt    336
   Tyr Arg Lys Gly Glu Asp Asp Met Gly Arg Val Tyr Gly Val Gln Gly
               100                 105                 110
   aga gct tgg gct aag cct gat ggt ggt cat att gac cag ttg aaa aag    384
   Arg Ala Trp Ala Lys Pro Asp Gly Gly His Ile Asp Gln Leu Lys Lys
           115                 120                 125
   att gtt gat gat ttg agc cgt ggc gtt gat gac cga ggt gaa att ctt    432
   Ile Val Asp Asp Leu Ser Arg Gly Val Asp Asp Arg Gly Glu Ile Leu
       130                 135                 140
   aac ttc tac aat ccg ggt gaa ttt cac atg ggg tgt ttg cgc cct tgc    480
   Asn Phe Tyr Asn Pro Gly Glu Phe His Met Gly Cys Leu Arg Pro Cys
   145                 150                 155                 160
   atg tac agc cat cat ttt tca ttg ctg ggt gat acc ttg tat ctc aac    528
   Met Tyr Ser His His Phe Ser Leu Leu Gly Asp Thr Leu Tyr Leu Asn
                   165                 170                 175
   agt act cag cgt tca tgt gat gtg ccc ttg ggg ttg aat ttc aac atg    576
   Ser Thr Gln Arg Ser Cys Asp Val Pro Leu Gly Leu Asn Phe Asn Met
               180                 185                 190
   gtg cag gtt tat gtg ttc ctt gcg ctg atg gca cag atc aca ggg aaa    624
   Val Gln Val Tyr Val Phe Leu Ala Leu Met Ala Gln Ile Thr Gly Lys
           195                 200                 205
   aag ccg ggc ttg gcg tat cac aag atc gtc aat gcg cac att tac caa    672
   Lys Pro Gly Leu Ala Tyr His Lys Ile Val Asn Ala His Ile Tyr Gln
       210                 215                 220
   gat caa ctc gaa ttg atg cgc gat gtg cag cta aaa cgt gag cca ttc    720
   Asp Gln Leu Glu Leu Met Arg Asp Val Gln Leu Lys Arg Glu Pro Phe
   225                 230                 235                 240
   cca gcg cct cag ttc cat atc aat cca aag att aaa aca ctg cag gat    768
   Pro Ala Pro Gln Phe His Ile Asn Pro Lys Ile Lys Thr Leu Gln Asp
                   245                 250                 255
   ttg gaa act tgg gtc act ttg gat gat ttt gac gtc acc gga tat cag    816
   Leu Glu Thr Trp Val Thr Leu Asp Asp Phe Asp Val Thr Gly Tyr Gln
   260 265 270
   ttc cac gat cct att caa tac ccg ttt tca gtc taa 852
   Phe His Asp Pro Ile Gln Tyr Pro Phe Ser Val
   275 280
<210> 2
   <211> 284
   <212> PRT
   <213> Vibrio cholerae
<400> 2

## Description

### Technical sector

The field of invention is that of Biotechnology and more specifically the generation of *Vibrio cholerae* vaccines and the methods of constructing them by using genetic engineering tools.

### Background of the invention.

A brief explanation on the terminology used through the text of the invention is listed below.

By ctxΦ virus is meant the particle of protein-coated DNA, produced by certain *Vibrio cholerae* strains, which is capable of transducing its DNA, comprising cholera toxin genes, to other *Vibrio cholerae* strains.

By cholera toxin is meant the protein responsible for the clinical symptoms of cholerae when produced by the bacteria.

By ctxΦ-encoded toxin genes are meant, in addition to cholera toxin genes, *zot* and *ace* genes, which code for the "zonula occludens toxin" and for the "accessory cholera enterotoxin", respectively.

For non-toxigenic strains of *Vibrio cholerae* it most be understood any strain devoid of the genes coding for the above toxins, which are as well, useful as vaccines but still produce an undesired reactogenic syndrome.

The term safe vaccine or safe strain refers to such strain lacking the residual reactogenicity of non-toxigenic strains of *Vibrio cholerae*.

By hemagglutinin/protease is meant the protein manifesting dual function, being one of them the ability to agglutinate erythrocytes from certain species and the other the property to degrade proteins such as mucin.

The term *cel*A refers to the nucleotide sequence coding for the endcglucanase A protein. This protein naturally occurs in *Clostridium thermocellum* strains and has a β(1-4) glucan-glucane hydrolytic activity able to degrade cellulose and its derivatives.

By Thymidylate synthase is meant the protein capable of catalyzing he reductive methylation of deoxyuracil monophosphate (dUMP) by N⁵-N¹⁰-methylene-tetrahydrofolate to yield 2,5 deoxythymidyne phosphate (dTMP) and dihydrofolate.

Substantially pure DNA is DNA that is free from both of the coding sequences immediately contiguous by the 5' or the 3' end of *thy*A coding sequence, in the naturally occurring genome of the microorganism from which the DNA of the invention is derived. The term thereof includes, for example, a recombinant DNA which is incorporated on a vector strain, cell line or plasmid, or which exists as a separate molecule (e.g., cDNA, restriction or PCR fragment). It also includes recombinant DNA molecules which are part of a hybrid gene encoding additional sequences.

Homologous sequences refers to DNA or protein sequences which share similar or identical residues being nucleotides or amino acids, respectively, in identical positions of two or more given strings. The greater the number of identical/similar residues in certain position, the greater the percent of identity/similarity between two them.

Clinical cholera is an acute diarrheal disease that results from an oral infection with the bacterium *Vibrio cholerae*. After more than 100 years of research on cholera there remains the need for an effective and safe vaccine. Humankind has witnessed seven pandemics of cholera; the former six were caused by strains of the classical biotype and the current seventh pandemic is characterized by the predominance of Vibrios belonging to El Tor Biotype. Recently, beginning in January cf 1991, this pandemic has extended to South America causing greater than 25 000 cases and over 2000 deaths in Peru, Ecuador, Colombia, and Chile. By November 1992, a new serogroup emerged in India and Bangladesh, the O139, showing a great epidemic potential that became a new cause of concern throughout the developing world. These recent experiences reinforce the need for effective cholera vaccines against disease caused by *V. cholerae* of serogroups O1 (El Tor) and O139.

Because convalescence to cholera is followed by an state of immunity lasting at least 3 years, much of the efforts in *Vibrio cholerae* vaccinology have been made to produce live, attenuated cholera vaccines, that closely mimic the disease in its immunization properties after oral administration, but do not result reactogenic to the individuals ingesting them. Vaccines of this type involve deletion mutations of all toxin genes encoded by the ctxϕ Vibrio-phage. See patents of Kaper, J. et al,; WO 91,18979 and Mekalanos, J., WO 95,18633).

The first vaccine to be assayed against cholera dates from 1885-1892. It was a traditional vaccine that comprised administration by parenteral route of "attenuated" vibrios. It resulted limited in efficacy and unacceptably reactogenic (Finkelstein R. A., International Symposium on Cholera on the America Continent. Sao Paulo, SP Brasil, 1992). Oral vaccination was tried first in 1892, using attenuated *Vibrio cholerae* strains. The results of this attempt were misinterpreted and the strategy immediately abandoned. Oral vaccination was later rescued in 1970-1980 at the Cente- for Vaccine Development of Maryland, USA, by using chemically mutagenized vibrios as immunizing agents. Reversion to virulence of these mutants impeded further spread of the strategy (Levine et al., Infect and Imm, N°2, 1984; Finkelstein et al., patent US 4,328,209) and prompted the researchers to generate genetically defined non-toxigenic mutants unable to revert. Although these mutants have shown to confer solid immunological protection against disease (Kaper J. B. and Levine M. Patents US 06,472,276 and 581,406), the essential drawback for their use is the high level of adverse reactions they produce in vaccinees (Levine et al., Infect. and Imm. Vol 56, N°1, 1988). According to these data the major issue to be overcome when producing an effective cholera vaccine is safety. Additionally, researches worldwide are currently concerned on the horizontal transfer of genetic information among bacteria, thus it is necessary to pay attention to this aspect when designing live bacterial vaccines, with the aim to ameliorate the environmental impact during vaccination. It is also necessary to achieve good levels of stability and immunogenicity.

A dead cholerae vaccine consisting of whole cells supplemented with the B subunit of cholera toxin is available (Holmgren et al., Current topics in Microbiology and Immunology, Vol. 146, 1989). Such vaccine is safe and effective but requires multiple doses to generate an immune response equivalent to that of a cholera infection and consequently is very expensive.

Another alternative for cholera vaccination is the recent licensed CVD103-HgR, a live cholera vaccine belonging to the classical biotype. It is safe, effective and cheap; however its protective efficacy against the current circulating El Tor and O'39 vibrios is not as good as against Classical vibrios (See patent USA, 5399494).

Other live vaccine candidates have been described in patent WO 95/18633. Such mutants represent all serotypes of the current pandemic, including the O139. They are safe, their production is cheap, and have been shown to be preliminary effective; however they are not as extensively tested as CVD103-HgR. All these candidates are protothrophic bacteria able to survive natural conditions of the environment. Additionally, although a procedure to obtain defined mutants is described in the document, the proposed candidates constitute non-motile spontaneous mutants. It has been proposed by the inventors that the non-motile nature of these vaccine candidates limits their ability to reach the enterocyte surface and avoid the elicilation of the reactogenic response characteristic of their parentals.

Robert et al., Vaccine, Vol,14, N°16, 1517-22, 1996, demonstrated the factibility of using the hemagglutinin/protease locus for the insertion of heterologous tags, without detriment of the colonizing capacity of vibrios. Colonization of the human small bowel by *Vibrio cholerae* is essential to induce a strong localized immune response of secreted IgA in the intestinal mucosa and to produce a long lasting immunity against cholera (Taylor et al., The Journal of Infectious Diseases, 1994, 170: 1518-23).

It has been wisely sustained by Dr. Mekalanos, that uptake of bacter a by Peyer's patches is a consequence of the colonization process that does not lead to reactogenicity and is considered an essential step in the localized immune response pathway. In contrast, the interaction of bacteria with enterocytes results in adverse reactions unacceptable for vaccine purposes (Mekalanos J. et al., Bull. Inst, Pasteur, 93: 255-262, 1995). According to this criteria mutations that interfere with the capacity of vibrios to reach the enterocytes are desired features of cholera vaccines.

### Description of the Invention

This invention issues a method of producing a vaccine strain suitable for oral administration to humans for inducing immunological protection against cholera characterised in that residual reactogenicity is removed or reduced to an acceptable level from a non-toxigenic mutant of *Vibrio cholerae* by disruption of the hemagglutinin/protease gene (*hap*) by with the marker gene *cei*A*,* and further characterised in that a defined and irreversible mutation is introduced into its *thy*A gene shown in SEQ. ID NOº 1 coding for Thymidilate synthase of *vibrio cholerae* whereby proliferation in natural ecosystems is limited. Vaccine strain belonging to either of the two serotypes of the El Tor Biotype or to the 0139 serotype of *Vibrio cholerae* are disclosed. Preferably this strain derives from a non-toxigenic mutant of *Vibrio cholerae*, obtained by means of genetic engineering tools and is tagged with the marker gene *cel*A within the *hap* locus. In the most preferred fashion the strain is 638, 1333, or L911.

Any vaccine strain of *Vibrio cholerae* belonging to existent biotypes and serotypes or any emergent serotype against which current vaccines are not effective can be genetically manipulated in the genes coding for HA/P and Thymidilate Synthase, to render a double mutant with improved environmental biosafety features. More preferably the mutant is a derivative of 81, 413 or SG251a, and in the most preferable way the strain is 638T or the *thy*A*⁻* derivatives of 1333 and L911. As well this contained version of vaccine strains is useful as an entity for antigen delivery to the mucosal immune system.

We have found such mutants to be low reactogenic in laboratory and/or clinical tests, although yet able to elicit strong immune responses when administered by oral route. As a result these hemagglutinin/protease defective mutants and their further derivatives share the desirable properties for a vaccine against cholera in humans.

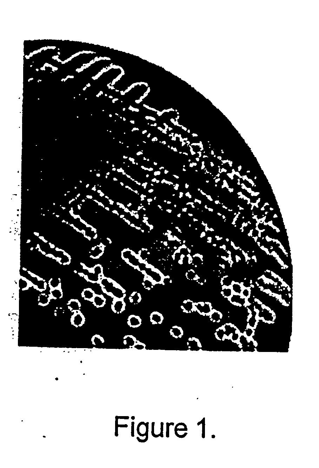

Additionally, disclosed is substantially pure DNA encoding the *thy*A gene for *Vibrio cholerae* Thymidilate Synthase. By *thy*A DNA is meant the DNA sequence shown in figure 1, SEQ. ID. No 1.

The construction of vaccine strains described herein involves replacement of the chromosomal gene coding for the wild type Hemagglutinin/Protease by a *cel*A disrupted allele in non-toxigenic mutants of *Vibrio cholerae* having deletec all genes encompassing the ctxϕ prophage. These mutants are genetically well defined and very stable in their proteolytic defect, showing no reversion detectable in 10⁹ cells. Said mutants are equally stable in their cellulolytic activity conferred by the *cel*A chromosomal marker, even after passage by the intestine of mice or humars, and further involves introducing a genetic deletion in the *thyA* gene shown in SEQ ID Nº 1 of the mutant, to obtain a thymine/thymidine auxotrophic derivative. The resultant triple mutant is genetically well defined and stable. As a consequence of this mutation the strain is provided with a resistance marker to the antibiotic trymethoprim, which is conditioned to the presence of thymine/thymidine. Such marker is unlikely to transform other bacteria by horizontal transfer due to its recessive nature.

Disclosed are non-toxigenic, genetically defined, stable, and safe mutants of *Vibrio cholerae* which are useful as a live, oral vaccines for inducing immunological protection against cholera in humans. When designing our non-reactogenic cholerae vaccine candidates we have stuck to the idea of reactogenicity as a consequence of interaction between *Vibrio cholerae* and enterocytes. We reasoned that inactivation of the major secreted protease, responsible for mucin degradation, would render a strain of *Vibrio cholerae* inefficient in penetration of the thick mucous layer of the enterocytes, but unchanged in the ability to reach the surface of the M cell and elicit a strong immune response. The major secreted, mucin degrading protease in strain C7258, C6706 and SG25-1 was found to be the soluble hemagglutinin/protease, a putative virulence factor described by Finkelstein, et al. Journal of Bacteriology, Vol. 173, No. 11, pp. 3311-3317, 1991. Paralleling the inactivation of the hemagglutinin/protease gene, a DNA fragment coding for the endoglucanase A of *Clostridium thermocellum* was inserted in the *Vibrio cholerae* chromosome. This mutation combined with the deletion of cholerae toxin genes of the ctxϕ genome resulted in tagged vaccine candidates with excellent properties as immur izing agents for vaccinating humans against cholera.

As an example, rather than as an interest to be limiting; the non-toxigenic mutants of *Vibrio cholerae* useful for constructing safe mutants defective in the expression of hemagglutinin/protease for vaccine purposes, are described in table 1 a. Non-toxigenic mutants are specifically characterized by the absence of al ctxφ coding sequences in their chromosome, and by the presence of a single RS1 element, the nucleotide sequence of which was confirm by DNA sequencing. The methods of producing non-toxigenic mutants of *Vibrio cholerae* are well described elsewhere (Archives of Medical Research, 27, No. 3, pp. 275-283).

**Table 1a. Starting strains for constructing HA/P defective mutants.**

| Vaccine candidate | Biotype/Serotype | Genotype |
|---|---|---|
| 81 | EI Tor/Ogawa | Δctxφ |
| 413 | EI Tor/Inaba | Δctxφ |
| SG25-1^{a} | O139 | Δctxφ |

| | | |
|---|---|---|
| All strains and the methods of making are described in Archives of Medical Research. Vo . 27. No. 3. pp. 275-283, 1996. 81 and 413 derive from C7258 and C6706, respectively; both of which are clinical isolates from Perú, 1991. SG25-1a is a derivative of the 0139 isolate SG25-1 from Calcutta, India, 1993. | | |

In a similar way, table 1b provides the hemagglutinin/protease mutants useful for constructing *thy*A defective derivatives with better features of biosafety.

**Table 1b. Hemagglutinin/Protease mutants useful for constructing thyA mutants.**

| Vaccine candidate | Relevant properties |
|---|---|
| 638 | 81 *hap::cel*A |
| 1333 | 413 *hap::cel*A |
| L911 | SG251a *hap::cel*A |

### Characterization of non-toxigenic vaccines with additional mutations intended to enhance their biosafety

### Serological characterization.

After any new mutation was introduced in vaccine strains described herein the derivative was demonstrated to retain the expected serotype. Cells were harvested from a plate, suspended in saline and immediately tested with Difco typing serum specific for Inaba, Ogawa or O139.

The major immune response elicited by vaccinees is directed against the LPS of the bacterium. All strains being issued in the present invention retained expression of the expected O-antigen as confirmed by serological tests. Additionally LPS profiles remained unchanged in polyacrylamide gel electrophoresis and Western blot.

### Infant mouse colonization assay.

The infant mouse colonization assay (Herrington et al, J. Exper. Med. 168:1487:1492, 1988) was used to asses the colonization properties of each mutant. An inoculum of 10⁵-10⁶ vibrios in a final volume of 50 µl was administered intragastrically to groups of at least five mice. After 18-24 hours at 30°C, mice were sacrificed, the intestine was dissected, homogenized and plated on bacteriological media containing appropriate supplements to support growth of mutants. Colonies thai grew after overnight incubation were tested for additional markers.

The ability of doubly and triply mutated (Δctxφ, HA/P)/(*thy*A⁻) strains of *Vibrio cholerae* to colonize the intestine of suckling mice can be observed in table 2. Strains 638, 1333 and 638T are well colonizers of the small bowell of mice. Colonization of gastrointestinal tract of infant mice is widely accepted to correlate well with colonization of the human gut, which is necessary to prime the mucosal immune system and to induce a strong secretory IgA response. Although L911 colonizes less eficiently than the rest it is still able to colonize.

It should be noted that 638T is a *thyA⁻* mutant of 638. The mutation introduced in this strain creates a thymine or thymidine dependence that reduces the ability of this strain to multiply in natural environments where free pyrimidines are usually absent from.

**Table 2. Colonizing capacities of hap::celA vaccine strains.**

| Strain | Input | Output | Biotype/Serotype | Relevant genotype |
|---|---|---|---|---|
| 638 | 1x10⁶ | 2.8x10⁵ | EI Tor/Ogawa | Δctxφ,*hap*::ce/A |
| 1333 | 2x10⁶ | 4.2x10⁵ | EI Tor/Inaba | Δctxφ,*hap*::ce/A |
| L911 | 1.2x10⁶ | 8x10³ | O139 | Δctxφ,*hap*::ce/A |
| 638T | 1.7x10⁶ | 6x10⁵ | EI Tor/Ogawa | Δctxφ,*hap*::ce/A, *thy*A⁻ |

### Detection of protease activity.

Milk-LB plates were used to detect proteolytic activities in supernatants of TSB-grown vibrios. For quantitation of protease activity the azocasein method was adapted from Ginther CL., Antimicrob. Agents Chemother. 15, 522-526, 1979. Briefly 1.1 ml of buffer (CaCl₂ 1mM; Tris 0.2M, pH 7.2; Azocasein 1%); were mixed with 200 µl of culture supernatant and incubated for 1hr at 37°C. The unreacted substrate was precipitated with 83 µl of TCA 40% for 10 min. followed by 10 min. centrifugation at 12000 rpm. The colored product remaining in solution was neutralized with NaOH and read at 450 nm. One unit of enzymatic activity was defined as the quantity of enzyme producing a net increase of one in the optical density of the sample in one hour of reaction.

The mutation introduced in the hemagglutinin/protease gene of strains disclosed herein, accounts for a reduction in 60-80% of the proteolytic activity as observed in mutants when compared to their non-toxigenic parents.

### Detection of vibrios expressing the endoglucanase A marker.

For CelA activity detection, vibrios were grown in LB plates for 24 hours, overlayed with CMC-indicator agar and incubated for 4 hours at 60°C. Endoglucanase A positive colonies were visualized after Congo Red staining and washing, as red colonies surrounded by a transparent halo in the red background of the plate. CMC-indicator agar was composed of 0.7% agarose, 0.5% CM-cellulose in phosphate-citrate buffer pH 6.3 and staining solution was 1% Congo Red in water.

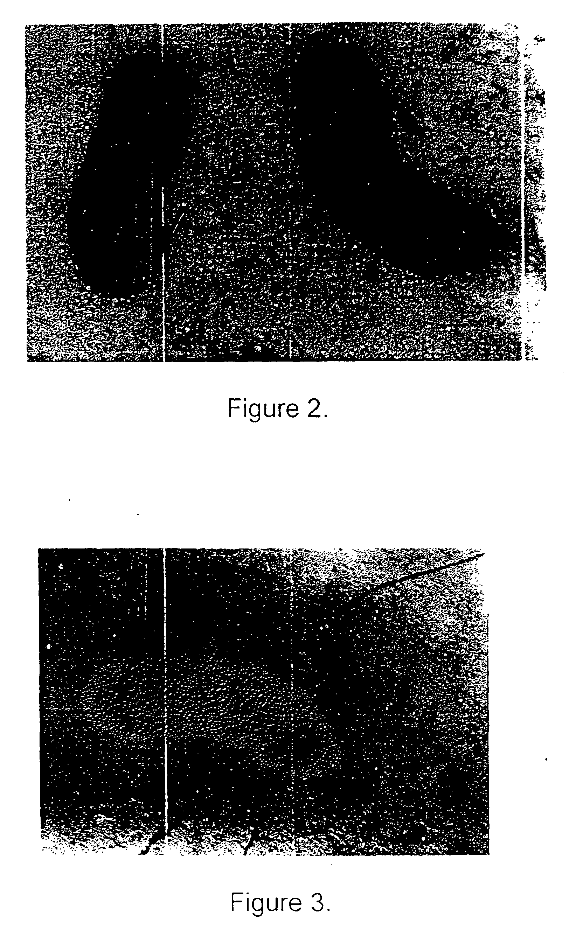

The *cel*A marker used to unequivocally distinguish the vaccine, is stably expressed and inherited in *Vibrio cholerae*. The appearance of tagged viorios can be observed in figure 2.

### Scoring for a thymidine auxotrohpy in vaccine strains generated by specific mutagenesis of thyA gene.

M9-salts supplemented with glucose as the carbon source and thymine/thymidine (200 µg/ml) were used to detect growth of *thy*A defective mutants impaired to grow in M9-salts glucose medium. LB plates supplemented with 200 µg/ml of thymidine were used to check for trimethropim resistance in the *thy*A mutants generated. The stability of thymidine auxotrophy was analyzed by replica-plating colonies from M9-glucose-thymidine to M9-Glucose plates.

Strains 638, 1333, and L911 are prototrophic bacteria able to grow in mineral salts employing glucose as the sole carbon source. Strain 638T requires addition of thymidine or thymine for growth in minimal medium. When one of these supplements is present 638T is resistant to up to 200 µg/ml of trimethoprim.

### Assay for motility.

Cells from single well isolated colonies were picked from a master plate aided with the tip of a platinum needle and inoculated by insertion (2-3 mm) into a motility agar plate (LB; agar 0.4%). The diameter each colony spreaded through soft agar was recorded at 24 hours of incubation at 30°C. The criteria for motility was as follows. A bacterial strain spreading 3 mm or less from the point of application was considered non-motile. A bacterial strain spreading >3 mm beyond the point of inoculation was considered motile.

All strains under the scope of this patent, but L911 were found to be motile. L911 is a derivative of SG251, which in our hands, resulted non-motile when assayed in motility agar.

### Assay for flagella

Motile and non-motile bacteria were assayed for the presence of flagella by electron microscopy. Briefly, *Vibrio cholerae* was grown for 4 hours at 37°C on colonization factor antigen agar (CFA, casaminoacids, 1 %; Yeast extract, 0.15%; Magnesium Sulphate, 0.05%; Manganesium Chloride, 0.005%), harvested and washed in saline (NaCl, 0.9%). Bacteria were negatively stained with 1% uranyl acetate for 3 min. and analyzed by transmission electron microscopy.

The flagellate nature of bacteria was also demonstrated for all vaccine strains as above, with greater emphasis on non-motile O139 strain L911 (figure 3). All strain described herein were shown to be flagellated, including the non-motile L911.

### Assays for TCP

Mutant strains were examined for the presence of toxin corregulated pilus on bacterial surface by immunogold electron microscopy. Cells were cultured as for the assay of flagella. Fresh harvested suspensions of vibrios were (10 µl) deoosited on a carbon coated nickel grid, fixed for 1 min. by exposing the grid to a 60 watts lamp and the excess of liquid removed on a filter pad. The grid was inverted in a drop of a TCP-specific sera diluted in saline, 1% BSA, 0.05% tween-80, incubated during 15 min. for reaction and washed in saline-BSA-Tween. Washed grids were incubated with the gold labeled conjugate diluted in the same buffer, let to stand for 15 min. and washed 3 times with saline-BSA-Tween. After washing, samples were stained during 1 min with a 1% ammonium molibdate solution.

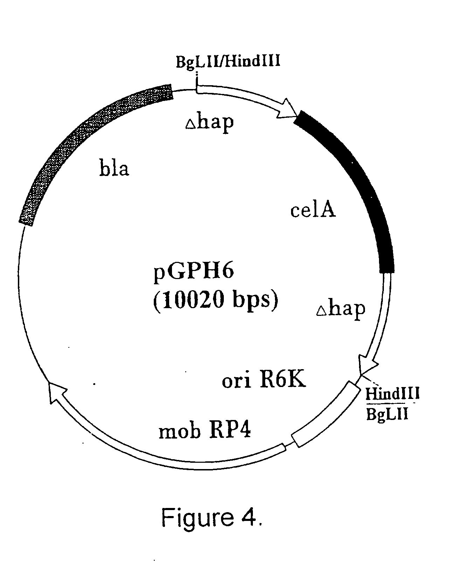

The most important features of cholera vaccines are safety and antigenicity. By genetic manipulation, *V. cholerae* strains are rendered safe, but care should be taken to retain their antigenicity unchanged. *Vibrio cholerae* strains disclosed in the preset patent application were evaluated for the expression of most prominent antigens in the bacterium. TCP was visualized on bacterial surface by immunogold electron microscopy. Alternatively, expression of this key colonization factor was detected by Western analysis. All strains analyzed (e.g. 638, 1333, L911 and 638T) produced normal levels of TCP protein and assembled it into large appendages on bacterial surface (figure 4).

### Live Vaccine strains

Strains 638, 1333, L911 and 638T described herein, can be used to achieve adequate immunological protection against cholerae in humans, provided the high rates of seroconversion they elicit and the low levels of adverse reactions they produce when orally administered to human volunteers. Depending upon relevant local epidemiology a single strain or combinations of them could be used for immunization.

### Culturability of vaccine strain

Vaccine strains were cultured in standard laboratory medium. Suplements of thymidine (200 µg/ml) were added when necessary for growth, but no supplements were included in the inoculum.

### Dosage

A single oral dose of live vaccine was administered to people in bicarbonate buffer (2%) after harvesting bacteria from a fresh plate in aseptic saline (NaCl 0.9%). Useful inoculum are 10⁷-10⁹ cells per immunizing dose. Alternatively more than one dose, separated 7-28 days apart, can be given to each subject. Preferably vibrios can be lyophilized in a formulation that preserves viability and mixed with bicarbonate prior to inoculation.

### Clinical testing of 638.

Strain 638 has been tested in human trials involving 42 volunteers aged 18-40. A summary of clinical findings after ingestion of strain 638 and placebo is shown in Table 3. All clinical manifestations observed were mild and of short duration. No statistical significance could be demonstrated between the inoculated and placebo groups with the present data. Gurgling and abdominal cramps were the reactions more frequently reported by volunteers irrespective of dose. Four volunteers developed mild diarrhea (grade 3). Three of these volunteers received the high dose and one the medium dose. One volunteer who received the high dose, had 5 loose stools (72 h after inoculation) and a total output of 680 g. Two volunteers had 2 loose stools 28 and 72 h after inoculation with total outputs of 220 and 500 g, respectively. The other volunteer had a single diarrheal output of 300 g 73 h after inoculation.

### Bacteriological isolation of vaccine strain.

As indicated in Table 4, strain 638 was recovered in 37 out of 42 volunteers inoculated (88 %). For the higher dose, excretion of the vaccine strain tend to peak at 72 h after inoculation. Three out of the 4 cases of diarrhea occurred at this time. Of the 37 volunteers that excreted vibrios, 12 excreted on at least 4 days, 19 on at least 3 days, and 28 on 2 days. The number of volunteers excreting strain 638 and the mean number of vibrios/ g stool declined in the lower dose. Vibrios isolated from the stool of volunteers produced endoglucanase A indicating that the *celA* reporter gene was stably maintained during growth in the human intestine. We conclude that strain 638 is a good colonizer of the human small bowel.

### Immune response to vaccine strain.

Strain 638 elicited a significant and consistent immune response in terms of serum vibriocidal antibodies, serum anti-Ogawa LPS IgG or IgA, and Ogawa LPS-specific IgA ASC (Tables 5 y 6). Although reciprocal GMT peaked 14 days after inoculation, seroconversion was attained on day 7 and titers remained high till day 28. Seroconversion rates, peak reciprocal GMT, and ELISA titers were dose-dependent. However, even at the lowest dose, strain 638 elicited a significant vibrioc dal antibody response compared to placebo. A significant proportion of the volunteers which experimented seroconversion developed relatively high (≥ 1024) vibriocidal titers (Table 6). The high percentage of responders in the ASC evaluation (Table 5) reflects an effective stimulation of mucosal immunity, mainly sIgA, by strain 638 in correspondence with the elevated anti-LPS IgA titers encountered 14 days after inoculation. One volunteer who ingested placebo seroconverted for anti-LPS IgG. This volunteer had very low pre-inoculation anti-LPS serum IgG which increased to the cutoff value at day 7 and remained constant thereafter. Another volunteer who ingested placebo reached the cutoff value of ASC. Similarly, this volunteer had a very low pre-inoculation number of LPS-specific ASC. We conclude that strain 638 elicits a significant immune response.

**Table 3. Frequency of occurrence of adverse reactions after ingestion of El Tor Ogawa candidate vaccine strain 638.**

| Symptom | Group of volunteers | | | | R.R.³ | Confidence interval⁴ | Probability (Fisher) |
|---|---|---|---|---|---|---|---|
| | Inoculated¹ | | Placebo² | | | | |
| | + | - | + | - | | | |
| Diarrhea | 4 | 38 | 1 | 13 | 1.33 | 0.16-10.96 | 0.6329 |
| Abdominal cramps | 13 | 29 | 2 | 12 | 2.17 | 0.56-8.44 | 0.1944 |
| Gurgling | 14 | 28 | 3 | 11 | 1.56 | 0.52-4.63 | 0.3143 |
| Heartburn | 6 | 36 | 2 | 12 | 1.00 | 0.23-4.40 | 0.6850 |
| Headache | 7 | 35 | 0 | 14 | - | - | 0.1163 |
| Vomiting | 1 | 41 | 0 | 14 | - | - | 0.7500 |

| Volunteers with diarrhea | | | | | | | |
|---|---|---|---|---|---|---|---|
| Mean diarrheal stool weight (range) | | | | | | 425 g (220-680) | |
| Mean number of diarrheal stools per ill volunteer (range) | | | | | | 2 (1-5) | |

| | | | | | | | |
|---|---|---|---|---|---|---|---|
| Notes: ¹N = 42, ²N = 14, ³Relative Risk, ⁴(95 %). | | | | | | | |

**Table 4. Recovery of Vibrio cholerae strain 638 from the stools of volunteers**

| Group of Volunteers | Volunteers excreting vaccine strain/total | | | | | | |
|---|---|---|---|---|---|---|---|
| | Time after inoculation (h) | | | | | Total | Mean CFU/g stool |
| | 24 | 48 | 72 | 96 | 120 | | |
| High dose (1-2 x 10⁹) | 7/29 | 10/29 | 16/29 | 15/29 | 10/29 | 28/29 | 4.4 x 10⁶ |
| Medium dose (2 x 10⁸) | 6/6 | 5/6 | 4/6 | 4/6 | 3/6 | 6/6 | 5.5 x 10⁶ |
| Low dose (4 x 10⁷) | 1/7 | 2/7 | 2/7 | 2/7 | 2/7 | 3/7 | 2.7 x 10⁵ |

**Table 5. Anti-LPS IgA ASC response in peripheral blood of volunteers following ingestion of Vibrio cholerae strain 638.**

| Dose | Positives (%) | Mean ASC per 10 PBMC (range) |
|---|---|---|
| High | 27/29(93.1) | 485 (0-4750) |
| Medium | 6/6 (100) | 377 (40-1285) |
| Low | 617 (85.7) | 5 (0-65) |
| Placebo | 1/14(7.1) | 371 (0-2040) |

**Table 6. Serum antibody responses in volunteers orally administered Vibrio cholerae El Tor Ogawa strain 638**

| | Group of volunteers | | | |
|---|---|---|---|---|
| Response | High dose | Medium dose | Low dose | Placebo |
| Vibriocidal antibodies | | | | |
| Seroconversion rate¹ (%) | 24/29 (82) | 5/6 (83) | 5/7 (71) | 0/14 (0) |
| GMT (range): | | | | |
| Pre-inoculation | 47 (0-160) | 32 (0-40) | 33 (0-40) | 37 (0-320) |
| Post-inoculation | 873 | 639 | 389 | 46 |
| peak [14 days] | (0-20480) | (0-2560) | (40-2560) | (3-320) |
| Responders with titers ≥ 1024 | 10/24 | 4/5 | 4/5 | 0 |

| Anti-Ogawa LPS IgG | | | | |
|---|---|---|---|---|
| Seroconversion rate² (%) | 23/29 (79) | 4/6(67) | 3/7 (44) | 1/14 (7) |
| Log reciprocal titer³ ± SD: | | | | |
| Pre-inoculation | 0.12 ± 0.25 | 0.12 ± 0.29 | 0 | 0.03 ± 0.12 |
| Post-inoculation | 1.86 ± 1.12 | 1.59 ± 1.49 | 1.07 ± 1.36 | 0.19 ± 0.57 |
| Peak [14 days] | | | | |

| Anti-Ogawa LPS IgA | | | | |
|---|---|---|---|---|
| Seroconversion rate ² (%) | 26/29(90) | 6/6(100) | 5/7 (71) | 0/14(0) |
| Log reciprocal titer ³ ± SD: | | | | |
| Pre-inoculation | 0.1 ± 0.37 | 0 | 0.27 ± 0.37 | 0.13 ± 0.39 |
| Post-inoculation | 2.43 ± 1.0 | 2.68 ± 0.48 | 1.96 ± 1.25 | 0.19 ± 0.53 |
| peak [14 -days] peak [14 days] | | | | |

| | | | | |
|---|---|---|---|---|
| Notes: ¹Number of volunteers with fourfold increase in titer/total, ²Number of volunteers with a twofold increase in titer/total, ³logarithm of the reciprocal arithmetic mean titer. Abbreviations: GMT, geometric mean titer; SD, standard deviation of the mean. | | | | |

### IV Examples

The examples described herein were conceived to illustrate rather than to limit the invention.

### Constructing safe vaccine candidates from non-toxigenic parentals.

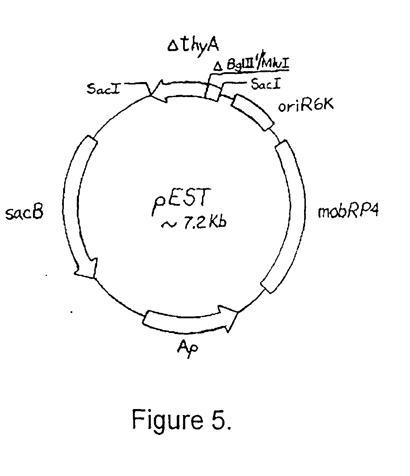

For constructing Hemagglutinin/Protease defective derivatives from non-toxigenic mutants described in table 1 a, each parental strain was equally processed. First, suicide vector pGPH6 (figure 5) containing the HA/P gene (*hap*) inactivated by insertion of reporter gene *cel*A was transferred from *E. coli* SM10λpir to the non-toxigenic mutant to produce an ampicillin resistant co-integrate. Second, Southern hybridization demonstrated the co-integrate to contain the insertionally inactivated *hap* gene (*hap::cel*A) and its wild type allele (*hap*) separated by vector DNA. Third, the above ampicillin-resistant co-integrate was allowed to segregate in antibiotic-free medium and ampicillin-sensitive colonies selected. Ampicillin-sensitive colonies designated 638, 1333 and L911, were characterized by Southern analysis and shown tc contain the *hap::cel*A mutant allele.

### Constructing 638T a Thymidilate Synthase defective derivative of Vibrio cholerae strains 638.

For constructing mutants of *Vibrio cholerae* which are defective in the expression of Thymidilate Synthase, its coding gene *thy*A was first cloned and sequenced. *Thy*A gene from *Vibrio cholerae* was first cloned by complementing a spontaneous trimethoprim-resistant thymidine-requiring mutant of strain 81, with a genomic library of strain C7258 constructed in pBR322. One clone was selected, purified and the insert brought to pUC19 which served as template to facilitate sequencing with universal primers. Nucleotide sequence of *thy*A gene and its predicted polypeptide of ThyA protein are shown in figure 1, seq. id No 1.

To construct a Thymidilate Synthase defective vaccine strain, an internal restriction fragment was deleted "in vitro" from the *thy*A open reading frame. Deletion comprised nucleotides between *Mlu*l and *Bgl*II restriction sites and removed DNA sequences coding for amino acid 7 to amino acid 105 of ThyA protein. The resultant gene construct was impaired in its ability to complement the *thy*A defect of spontaneous mutant *Vibrio cholerae* 815.

This fragment was cloned into the unique *Sac*l restriction site of pCVD442 to obtain pEST (figure 6). This plasmid was transferred from *E. coli* SM10λpir *Vibrio cholerae* strain 638 and an ampicillin-resistant co-integrate was selected. Southern analysis of the co-integrate demonstrated pEST was specifically integrated within the *thy*A gene. The ampicillin-resistant co-integrate was allowed to segregate in antibiotic-free medium supplemented with thymidine. Sucrose-resistant colonies were selected in the presence of thymidine. A thymidine-requiring, ampicillin-sensitive colony designated 638T, was characterized by Southern analysis and demonstrated to contain the dysfunctional *thy*A allele. pEST was used to construct 638T and will also oe useful for constructing 1333T, and L911 T or any additional defined *thy*A defective derivative other *V. cholerae* strain.

### A brief description of the drawings follows.

Figure 1. Nucleotide sequence of *thy*A gene and predicted amino acid sequence of encoded polypeptide.
Figure 2. Detection of cholerae vibrios tagged with *cel*A in plate assays employing CMC-indicator agar.
Figure 3. Detection of flagella on *Vibrio cholerae* mutant L911 of the O139 serogroup.
Figure 4. Detection of TCP on bacterial surface of 638T.
Figure 5. Schematic representation of suicide vector pGPH6 used to construct *hap::cel*A mutants of *V. cholerae*.
Figure 6. Schematic representation of suicide vector of pEST used to construct *thy*A mutants.

### Advantages

This invention provides us with an approach to create safer mutants of *Vibrio cholerae* by inactivation of the hemagglutinin/protease gene of non-toxigenic mutants. Such derivatives are useful as vaccines.

Safety and immunogenicity of strains 638, 1333 and L911 are similar to that presented in patent WO 95/18633. Additionally they are representative of all *Vibrio cholerae* strains circulating during the current pandemic and of the new O139 serogroup. Said strains are also tagged with a distinguishable marker to facilitate environmental sampling of the vaccine. All mutations introduced to construct the vaccines described herein are well defined as gene deletions or as gene insertions of known nature. This invention also discloses method to improve the environmental biosafety of live cholera strains to be used as oral vaccines. This improvement is attained by creating a defined mutation in the gene *thy*A which is also described herein.

Vaccine strain 638T is featured by its enhanced environmental biosafety. It has a thymidine auxotrophy that limits its proliferation in natural ecosystems, where free pyrimidines are scarce if existent.

Trimethoprim resistance conferred to *Vibrio cholerae* vaccine candidate 638T by its mutant *thy*A gene is unlikely to be transmitted into other bacteria due to its recessive nature. Additionally, resistance to trimethoprim is conditioned to the presence of thymine or thymidine in a culture.

Reacquisition of cholerae toxin genes or other DNA by means of horizontal gene transfer is superfluous in these strains since they do not proliferate out from the laboratory.

### SEQUENCE LIST

GENERAL INFORMATION
   APPLICANT
      NAME: Centro Nacional de Investigaciones Cientificas.
      STREET: Avenida 25, esquina a 158.
      CITY: Ciudad de La Habana.
      PROVINCE: Ciudad de La Habana.
      COUNTRY: Cuba.
      POST CODE: 12 100.
      TELEPHONE: 218066, extension 248.
      TELEFAX: 537 330497
      E-MAIL: "Rafael Fando" <Fando@biocnic.cneuro.edu.cu>
   TITLE: Novel *Vibrio cholerae* vaccine candidates and the methods of constructing.
   NUMBER OF SEQUENCES: 1
   CORRESPONDING ADDRESS
      ADDRESSEE: Consultores de Marcas y Patentes "CLAIMS".
      STREET: 14 #308 e/ 3ra y 5ta Ave. Miramar, Playa.
      CITY: Ciudad Habana.
      PROVINCE: Ciudad de La Habana.
      COUNTRY: Cuba.
      ZIP: 11 300.
   CURRENT APPLICATION DATA
      APPLICATION NUMBER: 142/97
      FILING DATE: December, 30, 1997.
   ATTORNEY/AGENT INFORMATION:
      NAME: Maria Lourdes Ruiz Sotolongo.
      REGISTRATION NUMBER: 5
INFORMATION FOR SEQ. ID. NO: 1
   SEQUENCE CHARACTERISTICS:
      LENGTH: 852 base pairs.
      TYPE: Nucleic acid.
      STRANDEDNESS: Single.
      TOPOLOGY: linear.
   TYPE OF MOLECULE: Genomic DNA.
   SOURCE:
      ORGANISM: *Vibrio cholerae*.
      STRAIN: C7258
   FEATURE:
      NAME**:** Coding sequence of the *thy*A gene.
      LOCALIZATION: 1-852
   FEATURE:
      NAME: Deduced polypeptide from the *thy*A coding sequence.
      LOCALIZATION: 1-284.
SEQUENCE DESCRIPTION: SEQ ID NO: 1

## Claims

1. A method of producing a vaccine strain suitable for oral administration to humans for inducing immunological protection against cholera **characterised in that** residual reactogenicity is removed or reduced to an acceptable level from a non-toxigenic mutant of *Vibrio cholerae* by disruption of the hemagglutinin/protease gene (*hap*) with the marker gene *cel*A*,* and further **characterised in that** a defined and irreversible mutation is introduced into its *thyA* gene shown in SEQ ID NO:1 coding for Thymidilate synthase of *vibrio cholerae,* whereby proliferation in natural ecosystems is limited.

2. The use of one or more *Vibrio cholerae* vaccine strains derived from non-toxigenic parentals in which the level of residual reactogenicity has been removed or reduced to an acceptable level by disruption of the hemagglutinin/protease gene (*hap*) with the marker gene *cel*A, in the manufacture of a medicinal preparation suitable for oral administration to humans for inducing immunological protection against cholera and wherein the said *Vibrio cholerae* vaccine strain or strains are further **characterised by** a defined and irreversible mutation introduced into their *thy*A gene shown in SEQ ID NO:1 coding for Thymidilate synthase whereby Proliferation in natural ecosystems is limited.

3. Use according to claim 2 wherein the said *Vibrio cholerae* vaccine strain or strains are admixed with a 2% bicarbonate buffer.

4. Use according to claim 2 or claim 3 of a lyophilised formulation.

5. A medicinal preparation with improved environmental biosafety suitable for administration to humans for inducing immunological protection against cholera comprising as an essential ingredient one or more *Vibrio cholerae* vaccine strains derived from a non-toxigenic parental, the strain or strains being **characterised by** an acceptable level of residual reactogenicity due to the presence of a dysfunctional hemagglutinin/protease gene (*hap*) resulting from disruption of the *hap* gene with the marker gene *cel*A and further **characterised by** the presence of a defined and irreversible mutation introduced into the *thy*A gene shown in SEQ ID NO:1 coding for Thymidilate synthase whereby proliferation in natural ecosystems is limited.

## Patentansprüche

1. Methode zur Herstellung eines Impfstoffkandidaten, welcher geeignet ist für die orale Verabreichung an Menschen, um einen immunologischen Schutz gegen Cholera zu induzieren, **dadurch gekennzeichnet, dass** die verbleibende Reaktogenität entfernt oder auf ein akzeptables Niveau vermindert wird von einem nicht-toxigenen Mutant der *Vibrio cholerae* durch die Spaltung des Hämagglutinin/Protease Gens *(hap)* mit dem Markierungsgen *celA,* und des Weiteren **dadurch gekennzeichnet, dass** eine definierte und irreversible Mutation in sein *thyA* Gen eingeführt wird, wie in SEQIDNO: 1 gezeigt, kodierend für die Thymidilat Synthase von *Vibrio cholerae,* wodurch die Proliferation in natürlichen Ökosystemen beschränkt ist.

2. Verwendung von einem oder mehreren *Vibrio cholerae* Impfstoffkandidaten, abgeleitet von nicht-toxigenen Ausgangsstämmen, in welchen das Niveau der verbleibenden Reaktogenität entfernt oder auf ein akzeptables Niveau vermindert wurde, durch die Spaltung des Hämagglutinin/Protease Gens *(hap)* mit dem Markierungsgen *celA,* in der Herstellung einer medizinischen Zubereitung, welche geeignet ist für die orale Verabreichung an Menschen, um einen immunologischen Schutz gegen Cholera zu induzieren, und wobei dieser *Vibrio cholerae* Kandidat oder diese Kandidaten weiter **gekennzeichnet sind durch** eine definierte und irreversible Mutation, welche in ihr *thyA* Gen eingeführt wird, wie in SEQIDNO: 1 gezeigt, kodierend für die Thymidilat Synthase, wodurch die Proliferation in natürlichen Ökosystemen beschränkt ist.

3. Verwendung nach Anspruch 2, wobei der *Vibrio cholerae* Impfstoffkandidat oder die Impfstoffkandidaten gemischt sind mit 2 % Bikarbonat Puffer.

4. Verwendung nach Anspruch 2 oder Anspruch 3 einer lyophilisierten Formulierung.

5. Medizinische Zubereitung mit verbesserter umweltbezogener Biosicherheit, geeignet für die Verabreichung an Menschen, um einen immunologischen Schutz gegen Cholera zu induzieren, welche als wesentlichen Bestandteil einen oder mehrere *Vibrio cholerae* Impfstoffkandidaten, abgeleitet von einem nicht-toxigenen Ausgangsstamm, umfassen, wobei der Kandidat oder die Kandidaten **gekennzeichnet sind durch** ein akzeptables Niveau der verbleibenden Reaktogenität infolge der Anwesenheit eines dysfunktionalen Hämagglutinin/Protease Gens *(hap)* resultierend aus der Spaltung des *hap* Gens mit dem Markierungsgen *celA,* und des Weiteren **gekennzeichnet durch** die Anwesenheit einer definierten und irreversiblen Mutation, welche in das *thyA* Gen eingeführt wird, wie in SEQIDNO: 1 gezeigt, kodierend für die Thymidilat Synthase, wodurch die Proliferation in natürlichen Ökosystemen beschränkt ist.

## Revendications

1. Procédé de fabrication d'un candidat vaccin approprié pour l'administration par voie orale aux êtres humains pour induire une protection immunologique contre le choléra **caractérisé en ce que** la réactogénicité résiduelle est enlevée ou réduite à un niveau acceptable d'un mutant non toxigénique de *Vibrio cholerae* par la disruption du gène hémagglutinine/protéase *(hap)* avec le gène marqueur *cel*A, et de plus **caractérisé en ce qu'**une mutation définie et irréversible est introduite dans le gène *thy*A*,* montré dans SEQIDNO: 1, codant pour la thymidilate synthase de *Vibrio cholerae,* par laquelle la prolifération dans les écosystèmes est limitée.

2. Utilisation d'un ou de plusieurs candidats vaccins *Vibrio cholerae* dérivés de souches parentales non toxigéniques dans lesquels le niveau de réactogénicité résiduelle a été enlevé ou réduit à un niveau acceptable par la disruption du gène hémagglutinine/protéase *(hap)* avec le gène marqueur *celA,* dans la fabrication d'une préparation médicinale appropriée pour l'administration par voie orale aux êtres humains pour induire une protection immunologique contre le choléra et où le candidat vaccin ou les candidats vaccins *Vibrio cholerae* sont de plus **caractérisés en ce qu'**une mutation définie et irréversible est introduite dans leur gène *thyA,* montré dans SEQIDNO: 1, codant pour la thymidilate synthase, par laquelle la prolifération dans les écosystèmes est limitée.

3. Utilisation selon la revendication 2 où le candidat vaccin ou les candidats vaccins *Vibrio cholerae* sont mélangés avec 2% de tampon bicarbonate.

4. Utilisation selon la revendication 2 ou revendication 3 d'une formulation lyophilisée.

5. Préparation médicinale avec une biosécurité environnementale améliorée appropriée pour l'administration aux êtres humains pour induire une protection immunologique contre le choléra comprenant en tant qu'ingrédient essentiel un ou plusieurs candidats vaccins *Vibrio cholerae* dérivés d'une souche parentale non toxigénique, où le candidat ou les candidats sont **caractérisés par** un niveau acceptable de réactogénicité résiduelle en raison de la présence d'un gène hémagglutinine/protéase (*hap*) dysfonctionnel qui résulte de la disruption du gène *hap* avec le gène marqueur *celA* et de plus **caractérisés par** la présence d'une mutation définie et irréversible introduite dans le gène *thyA,* montré dans SEQIDNO: 1, codant pour la thymidilate synthase, par laquelle la prolifération dans les écosystèmes est limitée.
